# EUROPÄISCHE PATENTSCHRIFT

(11) **EP 2 288 299 B1**
(45) Veröffentlichungstag und Bekanntmachung des Hinweises auf die Patenterteilung: **08.07.2015**
(21) Anmeldenummer: 08734949.4
(22) Anmeldetag: 01.04.2008
(51) Int. Cl.: A61B 17/22

(54) **DRAHTKORBEINHEIT MIT FIXIERSCHEIBENELEMENT**
WIRE BASKET UNIT HAVING FIXING DISK ELEMENT
ENSEMBLE PANIER EN FILS MÉTALLIQUES DOTÉ D'UN DISQUE DE FIXATION

(43) Veröffentlichungstag der Anmeldung: 02.03.2011
(73) Patentinhaber: Coloplast A/S, 3050 Humlebæk (DK)
(72) Erfinder: UIHLEIN, Bernhard, 72581 Dettingen (DE); TORCHIO, Gerard, Louis, Florent, F-91370 Verrières le Buisson (FR)
(86) Internationale Anmeldenummer: PCT/EP2008/002600
(87) Internationale Veröffentlichungsnummer: WO 2009/121379

(56) Entgegenhaltungen:
- WO-A-02/078632
- DE-A1- 10 150 399
- DE-A1-102005 030 010
- DE-C1- 10 117 836
- US-A1- 2006 058 813

## Beschreibung

Die Erfindung bezieht sich auf eine Drahtkorbeinheit, insbesondere für ein medizinisches Drahtkorbinstrument, mit einem ballonförmigem Drahtkorb aus mehreren Drahtsträngen, die sich zwischen einem vorderen und einem hinteren Drahtkorbende erstrecken und von einem oder mehreren Drahtstücken gebildet sind, und einem Fixierscheibenelement, an dem die Drahtstränge am vorderen Drahtkorbende unter Bildung eines vorderen Drahtkorbabschlusses fixiert sind, wobei das Fixierscheibenelement wenigstens zwei Durchführungsöffnungen aufweist.

Eine Drahtkorbeinheit dieser Art ist für ein medizinisches Instrument in der Offenlegungsschrift DE 101 50 399 A1 und in der WO 02/078632 offenbart. Derartige medizinische Instrumente werden beispielsweise als Steinfangkorb- und Polypektomieschlingeninstrumente verwendet. Bei diesen Anwendungen kann der ballonförmige Drahtkorb in eine Aufnahmehülse zusammenfaltend eingezogen und aus dieser wieder auffaltend herausbewegt werden. Dabei sollte eine bleibende Verformung der korbbildenden Drahtstränge beim Einziehen in die Aufnahmehülse vermieden und im Gebrauch z.B. beim Einfangen von Steinen in menschlichen oder tierischen Gewebehohlräumen die Gefahr von Gewebeperforationen minimiert werden. Um der letztgenannten Problematik zu begegnen, ist häufig eine Realisierung des ballonförmigen Drahtkorbs mit im Wesentlichen spitzenlosem Vorderende ("tipless basket") gewünscht. Zur Erfüllung dieser Anforderungen wird in der DE 101 50 399 A1 die Verwendung eines Ringelements oder eines gelochten Scheibenelements mit einer oder auch mehreren Durchführungsöffnungen vorgeschlagen, durch welche die Drahtstücke ein Mal hindurchgeschleift und unter Bildung einer Öse am Ringelement bzw. gelochten Scheibenelement fixiert sind. Bei Verwendung eines gelochten Scheibenelements ist dieses vorzugsweise mit umfangseitigen Einbuchtungen versehen, um dort Aufnahmeraum für die ösenbildenden Drahtstücke zu schaffen.

Der Erfindung liegt als technisches Problem die Bereitstellung einer Drahtkorbeinheit der eingangs genannten Art zugrunde, die gegenüber dem oben genannten Stand der Technik hinsichtlich der Drahtstrangfixierung durch das Fixierscheibenelement am vorderen Drahtkorbende weiter verbessert ist.

Die Erfindung löst dieses Problem durch die Bereitstellung einer Drahtkorbeinheit mit den Merkmalen des Anspruchs 1. Bei dieser Drahtkorbeinheit ist wenigstens ein erster der Drahtstränge von einem ersten Drahtstück gebildet, das durch eine erste der Durchführungsöffnungen des Fixierscheibenelements von hinten nach vorne und unter U-förmiger Umlenkung durch eine zweite der Durchführungsöffnungen des Fixierscheibenelements von vorne wieder nach hinten durchgeschleift und so am Fixierscheibenelement fixiert ist und das Fixierscheibenelement eine von einem Umfangsrandbereich zu einem Mittenbereich vorgewölbt gebogen Form aufweist.

Untersuchungen in der Praxis haben gezeigt, dass die so realisierte Drahtstrangfixierung an dem dazu mit mehreren Durchführungsöffnungen versehenen Fixierscheibenelement überraschend vorteilhaft hinsichtlich der eingangs angesprochenen Problempunkte und insbesondere auch hinsichtlich reproduzierbarem Zusammenfalten und Auffalten des ballonförmigen Drahtkorbs und guter Drahtkorbstabilität im aufgefalteten Zustand ist. Das U-förmige Durchschleifen des Drahtstücks durch zwei separate Durchführungsöffnungen des Fixierscheibenelements ermöglicht einerseits die für das Zusammen- und Auffalten des Drahtkorbs gewünschte und nötige Beweglichkeit der Drahtstrangfixierung am Fixierscheibenelement und verhindert andererseits eine zu lose Fixierung, die z.B. zu einem unerwünschten Verdrehen und damit Abkippen des Drahtkorbs führen kann.

In einer Weiterbildung der Erfindung nach Anspruch 2 sind wenigstens zwei der korbbildenden Drahtstränge von einem einteiligen Drahtstück gebildet, das am Fixierscheibenelement U-förmig umgelenkt und durch zwei zugehörige Durchführungsöffnungen durchgeschleift ist und sich von dort aus mit je einem Drahtstückabschnitt, die je einen der Drahtstränge des Drahtkorbs bilden, bis wenigstens zum hinteren Drahtkorbende nach hinten erstreckt. Auf diese Weise können von einem Drahtstück jeweils mindestens zwei der korbbildenden Drahtstränge bereitgestellt werden. In weiterer Ausgestaltung dieser Maßnahme sind gemäß Anspruch 3 mehrere Drahtstrangpaare in dieser Weise von jeweils einem einteiligen Drahtstück gebildet.

Die Durchführungsöffnungen können je nach Anwendungsfall geeignete Querschnittsformen aufweisen, von denen einige vorteilhafte Realisierungen gemäß der Erfindung im Anspruch 4 angegeben sind. Dabei können die Durchführungsöffnungen je nach Bedarf untereinander gleiche oder voneinander verschiedene Querschnittsformen aufweisen.

In weiterer Ausgestaltung der Erfindung sind gemäß Anspruch 5 die Durchführungsöffnungen mit gleichem Abstand von einem Mittelpunkt und in Umfangswinkelrichtung des Fixierscheibenelements äquidistant angeordnet. Dies ist z.B. für die Bereitstellung von Drahtkörben vorteilhaft, bei denen die Drahtstränge in Umfangsrichtung des Drahtkorbs äquidistant angeordnet sind.

In einer Ausgestaltung der Erfindung nach Anspruch 6 sind durch wenigstens eine der Durchführungsöffnungen wenigstens zwei Drahtstücke durchgeschleift. Diese Maßnahme kann insbesondere mit einer geeignet angepassten Querschnittsgestaltung dieser Durchführungsöffnung kombiniert sein, z.B. einer langlochförmigen Gestaltung. Dadurch kann bei Bedarf die Anzahl an Durchführungsöffnungen im Fixierscheibenelement kleiner als die Anzahl von korbbildenden Drahtsträngen sein.

In Ausgestaltung der Erfindung nach Anspruch 7 sind die Drahtstränge überkreuzungsfrei am Fixierscheibenelement fixiert. Dies kann z.B. hinsichtlich Erzielung gleicher Umlenkbedingungen und damit gleicher Fixierbedingungen für alle Drahtstränge in entsprechenden Anwendungsfällen von Vorteil sein. Alternativ kann auch eine sich überkreuzende Umlenkung wenigstens zweier Drahtstränge am Fixierscheibenelement vorgesehen sein.

In einer vorteilhaften Weiterbildung der Erfindung nach Anspruch 8 verläuft wenigstens ein Drahtstück in seinem Umlenkbereich, d.h. auf der dem eigentlichen Drahtkorbbereich entgegengesetzten, vorderen Seite des Fixierscheibenelements, in einer nicht-parallelen, d.h. verdrehten Längsebene verglichen mit der Längsebene, in welcher wenigstens ein zugehöriger Drahtstrang im korbbildenden Abschnitt zwischen vorderem und hinterem Drahtkorbende verläuft. Es zeigt sich, dass diese Fixierungsmaßnahme hinsichtlich ausreichender Beweglichkeit der Fixierstelle einerseits und ausreichender Stabilität, insbesondere Verdrehsicherheit, der Fixierstelle andererseits sehr günstig ist.

In der Erfindung weist das Fixierscheibenelement eine zu seinem Mittenbereich hin vorgewölbte Form auf, z.B. eine Kugelschalenform. Diese Gestaltungsmaßnahme kann positive Auswirkungen auf die gewünschte Beweglichkeit der Fixierstelle für den jeweiligen Drahtstrang haben und einen an die Ballonform des daran anschließenden Drahtkorbbereichs anpassenden Übergang des vorderen Drahtkorbabschlusses bereitstellen.

Vorteilhafte Ausführungsformen der Erfindung sind in den Zeichnungen dargestellt und werden nachfolgend beschrieben. Hierbei zeigen:
- Fig. 1: eine Seitenansicht einer medizinischen Drahtkorbeinheit mit ballonförmigem Drahtkorb aus in einen Schaftbereich weitergeführten Drahtsträngen und mit vorderseitig abschließendem Drahtfixierelement,
- Fig. 2: eine Seitenansicht einer Variante der medizinischen Drahtkorbeinheit von Fig. 1, bei der die drahtkorbbildenden Drahtstränge am hinteren Drahtkorbende in einer Aufnahme gefasst sind,
- Fig. 3: eine Draufsicht auf den vorderen Bereich des Drahtkorbs von Fig. 1 oder 2 mit einem planen Vierloch-Fixierscheibenelement als Drahtfixierelement,
- Fig. 4: eine Seitenansicht des vorderen Drahtkorbbereichs von Fig. 3 längs eines Pfeils IV in Fig. 3,
- Fig. 5: eine Schnittansicht längs einer Linie V-V von Fig. 3,
- Fig. 6: eine Schnittansicht längs einer Linie VI-VI von Fig. 3,
- Fig. 7: eine Draufsicht entsprechend Fig. 3 für eine Variante mit aus zwei einzelnen Drahtsträngen bestehendem Drahtkorb,
- Fig. 8: eine Schnittansicht längs einer Linie VIII-VIII von Fig. 7,
- Fig. 9: eine Schnittansicht des vorderen Drahtkorbbereichs von Fig. 3 in einem in eine Aufnahmehülse eingezogenen Drahtkorbzustand,
- Fig. 10: eine Längsschnittansicht eines modifizierten, gekrümmten Fixierscheibenelements, nach der Erfindung,
- Fig. 11: eine Draufsicht auf einen gegenüber Fig. 3 modifizierten vorderen Drahtkorbabschluss mit sich überkreuzend durchgeschleiften Drahtstücken,
- Fig. 12: eine Draufsicht auf ein gegenüber demjenigen von Fig. 3 modifiziertes Fixierscheibenelement mit sechs Durchführungsöffnungen,
- Fig. 13: eine Draufsicht auf das Fixierscheibenelement von Fig. 12 mit drei durchgeschleiften Drahtstücken,
- Fig. 14: eine Ansicht entsprechend Fig. 12 für ein modifiziertes Fixierscheibenelement mit vier langlochartigen Durchführungsöffnungen,
- Fig. 15: die Ansicht von Fig. 14 mit zwei durchgeschleiften Drahtstücken,
- Fig. 16: eine Ansicht entsprechend Fig. 12 für ein weiteres modifiziertes Fixierscheibenelement mit zwei langlochförmigen Durchführungsöffnungen,
- Fig. 17: die Ansicht von Figur 16 mit zwei durchgeschleiften Drahtstücken,
- Fig. 18: eine Ansicht entsprechend Fig. 12 für ein weiteres modifiziertes Fixierscheibenelement mit vier sektorförmigen Durchführungsöffnungen und
- Fig. 19: die Ansicht von Figur 18 mit zwei durchgeschleiften Drahtstücken.

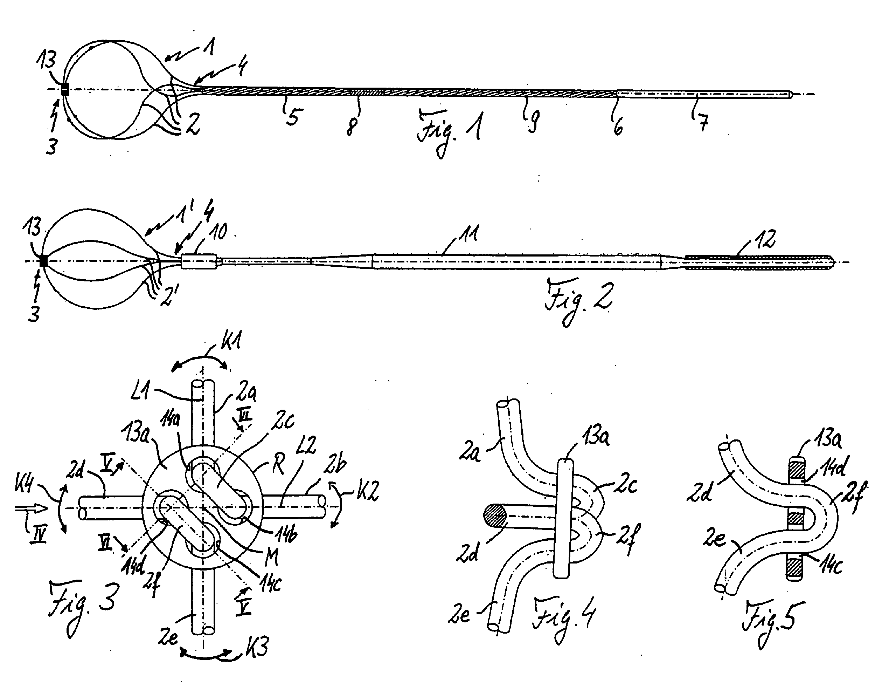

Die Fig. 1 und 2 zeigen zwei Drahtkorbeinheiten, wie sie in medizinischen Instrumenten eingesetzt werden können, insbesondere in Steinfangkorb- oder Polypektomieschlingeninstrumenten. Die Drahtkorbeinheit von Fig. 1 weist einen ballonförmigen Drahtkorb 1 aus vier Drahtsträngen 2 auf, die sich von einem vorderen Drahtkorbende 3 unter Bildung des ballonförmigen Drahtkorbs 1 zu einem hinteren Drahtkorbende 4 erstrecken und in verdrillter Form unter Bildung eines vorderen Schaftabschnitts 5 nach hinten bis zu einer Koppelstelle 8 weiter geführt sind, an der sie durch eine Schweißverbindung mit einem anschließenden mittleren verdrillten Schaftabschnitt 9 verbunden sind, der unter Fortsetzung dieser Drahtstränge oder aus eigenen Drahtsträngen aufgebaut sein kann. Der mittlere Schaftabschnitt 9 geht an einer Übergangsstelle 6 in einen hinteren Schaftabschnitt 7 über, der von einem massiven Kerndrahtmaterial gebildet ist.

Die Drahtkorbeinheit von Fig. 2 weist analog vier Drahtstränge 2' auf, die sich zwischen vorderem Drahtkorbende 3 und hinterem Drahtkorbende 4 unter Bildung eines ballonförmigen Drahtkorbs 1' erstrecken, wobei in diesem Ausführungsbeispiel die Drahtstränge 2' an ihrem hinteren Ende in einer Aufnahmehülse 10 gefasst sind. Bei dieser Drahtkorbeinheit ist der Schaft von einem massiven Kerndraht 11 gebildet, der über jeweils einen konischen Abschnitt in einem vorderen, an die Aufnahmehülse 10 anschließenden Bereich und in einem hinteren Bereich gegenüber einem mittleren Bereich verjüngt ist. Über den hinteren verjüngten Bereich des Kerndrahts 11 ist eine Ummantelung 12 aufgebracht.

Vorliegend ist primär die Gestaltung der Drahtkorbeinheit am vorderen Drahtkorbende 3 von Interesse, an welchem die korbbildenden Drahtstränge 2, 2' zusammengeführt und an einem Fixierelement 13 fixiert und dadurch miteinander verbunden sind. Zur Erzielung der gewünschten Ballonform für den Drahtkorb 1, 1' und eines möglichst spitzenlosen ("tipless") vorderen Drahtkorbabschlusses 3 ist es gewünscht, dass die Drahtstränge 2, 2' möglichst nahe am Fixierelement 13 in im Wesentlichen radialer Richtung auseinander laufen, bevor sie dann mit zunehmender Axialkomponente bis zum maximalen Korbdurchmesser verlaufen und von dort wieder bis zum hinteren Korbende 4 zusammen laufen. Das Fixierelement 13 hat zudem die Funktion, die Drahtstränge 2, 2' einerseits ausreichend beweglich und andererseits nicht zu lose zu fixieren. Die Beweglichkeit der Fixierung ist zur Gewährleistung einer gewünschten Elastizität des Drahtkorbs 1, 1' gefordert, insbesondere um in den besagten medizinischen Anwendungen die Drahtkorbeinheit samt Drahtkorb 1, 1' unter Zusammenfalten desselben in eine Aufnahmehülse einziehen zu können und sie so in einen menschlichen oder tierischen Gewebekanal einbringen zu können. Aus der Aufnahmehülse wird dann die Drahtkorbeinheit im Gebrauch vorgeschoben, bis der Drahtkorb 1, 1' wieder freiliegt und durch die Eigenelastizität der Drahtstränge 2, 2' seinen in den Fig. 1 und 2 gezeigten, aufgefalteten Funktionszustand einnimmt. Eine nicht zu lose Fixierung am Fixierelement 13 stabilisiert den aufgefalteten Drahtkorb 1, 1' und verhindert insbesondere ein unerwünschtes Verdrehen und damit Abkippen des Drahtkorbs 1, 1'.

Die Erfindung zeichnet sich durch eine besondere Realisierung des Fixierelements 13 und der Fixierung der Drahtstränge 2, 2' an selbigen aus, um diese Anforderungen bestmöglich zu erfüllen. Dazu ist erfindungsgemäß als Fixierelement 13 ein Fixierscheibenelement vorgesehen, das mit mehreren Durchführungsöffnungen versehen ist. Im Übrigen kann das Fixierscheibenelement von beliebiger Form sein, z.B. kreisförmig, oval, drei- oder mehreckig etc. Seine Dicke, d.h. Abmessung in axialer Richtung, ist vorzugsweise um ein Mehrfaches geringer als seine Querabmessung.

In den Fig. 3 bis 6 ist ein erstes Beispiel eines solchen Fixierscheibenelements (13a, 13b) gezeigt, wie es als Fixierelement 13 für die Drahtkorbeinheit von Fig. 1 oder von Fig. 2 oder für eine andere Drahtkorbeinheit mit ballonförmigem Drahtkorb einsetzbar ist. Wie gezeigt, weist das in diesem Fall kreisscheibenförmige Fixierscheibenelement (13a, 13b) vier Durchführungsöffnungen 14a, 14b, 14c, 14d auf, die im gezeigten Beispiel in äquidistantem 90°-Winkelabstand mit etwa gleichem Abstand von einem Mittelpunkt M und dem Scheibenrand R eingebracht sind. Ein erster Drahtstrang 2a und ein zweiter Drahtstrang 2b sind von einem ersten einteiligen Drahtstück gebildet, das unter Bildung eines U-förmigen Umlenkbereichs 2c durch zwei benachbarte Durchführungsöffnungen 14a, 14b durchgeschleift ist, d.h. das Drahtstück bildet vom hinteren Drahtkorbende kommend den ersten Drahtstrang 2a, ist dann von hinten nach vorne durch die Durchführungsöffnung 14a im Fixierscheibenelement (13a, 13b) durchgeschleift, biegt mit dem U-förmigen Abschnitt 2c an der Vorderseite des Fixierscheibenelements (13a, 13b) in die zweite Durchführungsöffnung 14b um, gelangt durch diese hindurch und erstreckt sich dann unter Bereitstellung des zweiten Drahtstrangs 2b wieder zum hinteren Drahtkorbende. Analog sind ein dritter Drahtstrang 2b und ein vierter Drahtstrang 2e des Drahtkorbs einteilig von einem zweiten Drahtstück gebildet, das mit einem U-förmigen Umlenkbereich 2f durch die beiden anderen benachbarten Durchführungsöffnungen 14c, 14d durchgeschleift ist.

Dies hat zur Folge, dass jedes der beiden einteiligen Drahtstücke jeweils zwei benachbarte, in Korbumfangsrichtung um 90° versetzte Drahtstränge 2a, 2b bzw. 2d, 2e bereitstellt. Außerdem verlaufen die beiden Umlenkbereiche 2c, 2f der Drahtstücke an der Vorderseite des Fixierscheibenelements 13a überkreuzungsfrei und in parallelen Längsebenen, wie der Schnittebene von Fig. 5, die nicht-parallel zu den Längsebenen sind, in denen die Drahtstränge 2a, 2b, 2d, 2e in ihrem korbbildenden Bereich verlaufen, d.h. einer Längsebene L1, in der die korbbildenden Abschnitte der Drahtstränge 2a und 2e liegen, und einer Längsebene L2, in der die korbbildenden Abschnitte der Drahtstränge 2b und 2d liegen. Im gezeigten Beispiel verlaufen die Drahtstücke im Umlenkbereich 2c, 2f unter einem Winkel von ca. 45° zu diesen beiden Längsebenen L1, L2, in denen die vier korbbildenden Abschnitte der Drahtstränge 2a, 2b, 2d, 2e liegen, wie aus Fig. 3 ersichtlich. Je nach Gestaltung und Positionierung der Durchführungsöffnungen sind selbstverständlich auch beliebige andere Orientierungen des oder der U-förmigen Umlenkbereiche der Drahtstränge bzw. Drahtstücke relativ zum Drahtstrangverlauf im korbbildenden Bereich möglich.

Zwar suggerieren die Fig. 4 bis 6 das Vorhandensein eines gewissen Spitzenbereichs, es handelt sich hierbei aber um schematische und nicht maßstäbliche Darstellungen ohne Bezugnahme zur Ausdehnung des gesamten Drahtkorbs. In realen Ausführungsbeispielen ist die axiale Ausdehnung des vorderen Drahtkorbabschlusses mit dem Fixierscheibenelement 13a und den U-förmigen Drahtumlenkbereichen 2c, 2f um ein Vielfaches bzw. eine oder mehrere Größenordnungen geringer als der Drahtkorbdurchmesser. Solche Ausführungen werden üblicherweise als spitzenlos bzw. quasi spitzenlos bezeichnet.

Versuche in der Praxis haben gezeigt, dass sich mit dieser Art der Fixierung der korbbildenden Drahtstränge 2a, 2b, 2d, 2e am Fixierscheibenelement 13a unter Bildung eines annähernd spitzenlosen vorderen Drahtkorbabschlusses die oben erwähnten Anforderungen hinsichtlich ausreichender Beweglichkeit einerseits und ausreichender Stabilität und Verdrehsicherheit der vorderseitigen Drahtstrangfixierung und damit des Drahtkorbs insgesamt überraschend gut erfüllen lassen. Dies wird besonders auch der Eigenschaft zugeschrieben, dass jeder der Drahtstränge 2a, 2b, 2d, 2e nicht nur durch eine Durchführungsöffnung 14a, 14c am Fixierscheibenelement 13a durchgesteckt gehalten ist, sondern sich zusätzlich unter Umlenkung an der Vorderseite des Fixierscheibenelements 13 durch eine zweite Durchführungsöffnung 14b, 14d durchgeführt gehalten ist.

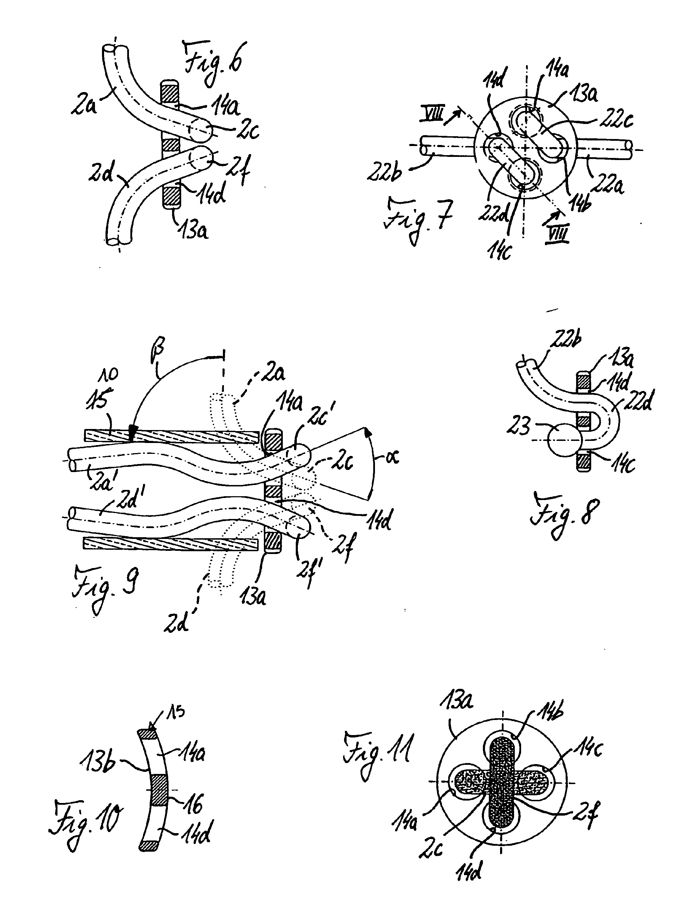

Im gezeigten Beispiel geht dabei jeder Drahtstrang in den vom selben Drahtstück einteilig bereitgestellten weiteren Drahtstrang über. In einer alternativen Ausführungsform endet der Drahtstrang einzeln nach Durchführen durch seine zweite Durchführungsöffnung z.B. unter Bildung eines verdickten Drahtendes, das gegen Herausgelangen aus der Durchführungsöffnung sichert. In diesem Fall sind dann zwei Durchführungsöffnungen für den einzelnen Drahtstrang erforderlich. Die Fig. 7 und 8 veranschaulichen ein entsprechendes Ausführungsbeispiel, bei dem der Drahtkorb aus zwei einzelnen Drahtsträngen bzw. Drahtstücken 22a, 22b als Drahtschlinge gebildet ist, indem die beiden korb- bzw. schlingenbildenden Drahtstränge 22a, 22b gegeneinander um 180° um die Korb- bzw. Schlingenlängsachse versetzt angeordnet sind.

Ein erstes Drahtstück 22a erstreckt sich vom hinteren Drahtkorb- bzw. Drahtschlingenende unter Bildung einer ersten Schlingenhälfte zum Fixierscheibenelement (13a, 13b) das hier identisch zu demjenigen des Ausführungsbeispiels der Fig. 3 bis 6 gewählt ist, durch eine erste 14b von dessen vier Durchführungsöffnungen 14a bis 14d hindurch, ist an der Vorderseite des Fixierscheibenelements (13a, 13b) mit einem U-förmigen Abschnitt 22c umgelenkt und durch eine zweite 14a der Durchführungsöffnungen 14a bis 14d des Fixierscheibenelements (13a, 13b) wieder nach hinten durchgesteckt, um dort an der Rückseite des Fixierscheibenelements (13a, 13b) mit einem gegen Herausbewegen sichernden verdickten Drahtende abzuschließen. In gleicher Weise erstreckt sich das zweite Drahtstück 22b vom hinteren Drahtkorbende unter Bildung der zweiten Schlingenhälfte nach vorn zum Fixierscheibenelement (13a, 13b) und dort, wie aus Fig. 8 genauer zu erkennen, durch eine dritte 14d der Durchführungsöffnungen 14a bis 14d hindurch, um an der Vorderseite des Fixierscheibenelements (13a, 13b) mit einem U-förmigen Umlenkbereich 22d durch die vierte 14c der Durchführungsöffnungen 14a bis 14d hindurch wieder zur Rückseite des Fixierscheibenelements (13a, 13b) zurückgeführt zu werden, wo es mit einem kugelförmig verdickten Drahtende 23 abschließt, dessen Außendurchmesser größer als der Durchmesser der zugehörigen Durchführungsöffnung 14c ist und dadurch gegen Herausbewegen des Drahtstücks 22b aus den zugehörigen Durchführungsöffnungen 14c, 14d sichert. Es versteht sich, dass in dieser Systemausführung mit jeweils einzeln am Fixierscheibenelement fixiertem Drahtstrang Drahtkorbeinheiten mit einer beliebigen, auch ungeraden Anzahl von drahtkorbbildenden Drahtsträngen realisierbar sind, z.B. für Drahtkörbe aus drei oder fünf Drahtsträngen.

In weiteren alternativen Ausführungsbeispielen können Mischformen vorgesehen sein, bei denen ein oder mehrere Drahtstrangpaare von jeweils einem Drahtstück bereitgestellt werden und wenigstens ein Drahtstrang einzeln von einem anderen Drahtstück bereitgestellt wird. In noch weiteren alternativen Ausführungsformen können auch mehr als zwei korbbildende Drahtstränge von einem einteiligen Drahtstück bereitgestellt werden, indem dieses Drahtstück am hinteren Drahtende einmal oder mehrmals um 180° umgebogen ist und jeweils wieder zum vorderen Drahtkorbende verläuft.

Die erwähnte Verdrehsicherheit ist für das Beispiel der Fig. 3 bis 6 in Fig. 3 mit Kipppfeilen K1, K2, K3, K4 symbolisiert. Die geschilderte Drahtstrangfixierung am Fixierscheibenelement 13a hat zur Folge, dass der Drahtkorb eine vergleichsweise hohe Steifigkeit gegenüber Biege- bzw. Verdrehbelastungen auf die Drahtstränge 2a bis 2d in dieser Pfeilrichtung aufweist, d.h. gegen Verdrehbelastungen auf die Drahtstränge 2a bis 2d quer zu ihrer Längserstreckung im korbbildenden Bereich. Diese Steifigkeit und damit Stabilität des Drahtkorbs ist insbesondere deutlich höher als bei einer Alternativlösung, bei der jeder Drahtstrang nur an einer einzigen zugehörigen Durchführungsöffnung eines entsprechenden Fixierscheibenelements gehalten ist. Sie ist auch deutlich höher als bei den eingangs erwähnten herkömmlichen Lösungen mit einem Ringelement als Fixierelement. Zudem bedarf es durch die vorliegende, erfindungsgemäße Lösung auch keiner Ösenbildung zum Fixieren der Drahtstränge am Fixierscheibenelement, wie dies bei den entsprechenden eingangs erwähnten herkömmlichen Lösungen mit gelochtem Fixierscheibenelement realisiert ist.

Dennoch bleibt für die erfindungsgemäße Drahtkorbeinheit eine Beweglichkeit der Drahtstrangfixierung am Fixierscheibenelement (13a, 13b) erhatten, wie sie benötigt wird, um den Drahtkorb in eine Aufnahmehülse einzuziehen und dabei zusammenzufalten. In Fig. 9 ist dies für das Ausführungsbeispiel der Fig. 3 bis 6 in einer der Fig. 6 entsprechenden Ansicht veranschaulicht. Durch das Einziehen in eine nur mit ihrem vorderen Teil gezeigte Aufnahmehülse 10 faltet sich der Drahtkorb zusammen, d.h. die einzelnen Drahtstränge 2a, 2b, 2d, 2e werden im korbbildenden Bereich zusammengedrückt. Die charakteristische Drahtstrangfixierung am Fixierscheibenelement (13a, 13b) ermöglicht zusammen mit der Eigenelastizität der Drahtstränge 2a, 2b, 2d, 2e dieses Zusammenfalten des Drahtkorbs in vorteilhafter Weise.

Wie aus Fig. 9 zu erkennen, sind die Durchführungsöffnungen 14a bis 14d im Fixierscheibenelement mit etwas größerem Durchmesser als der Durchmesser der Drahtstränge 2a, 2b, 2d, 2e eingebracht, so dass sich die durchgeschleiften Drahtstücke mit ihren Umlenkbereichen 2c, 2f in gewissem Maß in den Durchführungsöffnungen 14a bis 14d bewegen und dadurch der Zusammenfalt- bzw. Auffaltbewegung, welche die Drahtstränge 2a, 2b, 2d, 2e in ihrem korbbildenden Bereich erfahren, nachgiebig etwas folgen können. Zur Illustration ist in Fig. 9 punktiert der Verlauf der beiden Drahtstränge 2a und 2d im aufgefalteten Drahtkorbzustand gemäß Fig. 6 vergleichend zum mit durchgezogenen Linien dargestellten Verlauf dieser beiden Drahtstränge in ihrem korbbildenden Bereich 2a', 2d' und in ihrem Umlenkbereich 2c', 2f wiedergegeben. Wie daraus ersichtlich, bewegen sich die beiden Umlenkbereiche 2c, 2f beim Zusammenfalten des Drahtkorbs in einer Art Kippdrehbewegung um einen Kippwinkel α von beispielsweise ca. 45° etwas radial nach außen und erleichtern so das radiale Zusammenfalten der Drahtstränge im korbbildenden Bereich, das eine korrespondierende zusammenfaltende Kippdrehbewegung jedes Drahtstranges 2a, 2b, 2d, 2e in seinem hinten an das Fixierscheibenelement 13a angrenzenden Abschnitt um einen Faltwinkel β von ca. 90° beinhaltet. Dabei dienen die Durchführungsöffnungen 14a bis 14d in gewisser Weise als Drehpunkte für diese elastische Drahtstrangbewegung und lassen ein erforderliches Bewegungsspiel zu. Auf diese Weise ermöglicht die Beweglichkeit der Drahtfixierung zusammen mit der Eigenelastizität und der Anordnung der Drahtstränge 2a, 2b, 2d, 2e ein optimales Zusammenfalten des Drahtkorbs 1. Fig. 10 veranschaulicht die erfindungsgemäße Variante des im Beispiel der Fig. 3 bis 6 planen Fixierscheibenelements (13a, 13b). Diese Variante beinhaltet eine z.B. kugelschalenförmig gewölbte Gestaltung eines entsprechend modifizierten Fixierscheibenelements (13a, 13b), das im Übrigen demjenigen der Fig. 3 bis 6 entspricht, insbesondere was das Einbringen der vier Durchführungsöffnungen 14a bis 14d betrifft, so dass bis auf diesen Unterschied auf dessen obige Beschreibung verwiesen werden kann. Speziell weist dieses Fixierscheibenelement (13a, 13b) eine von einem Umfangsrandbereich 15 zu einem Mittenbereich 16 distal, d.h. nach vorn, vorgewölbte Form auf, was sich positiv auf die Beweglichkeit der wie beim Beispiel der Fig. 3 bis 6 in den Durchführungsöffnungen 14a bis 14b fixierten Drahtstränge auswirken kann. Außerdem verbeseit diese Formgebung des Fixierscheibenelements (13a, 13b) dessen Formanpassung an den sich nach hinten, d.h. in Fig. 10 nach links, anschließenden Drahtkorbbereich, da das Fixierscheibenelement (13a, 13b) auf diese Weise eine zum anschließenden Drahtkorbbereich gleichsinnige Krümmung aufweist.

Fig. 11 zeigt eine zum Ausführungsbeispiel der Fig. 3 bis 6 alternative Durchschleifung der beiden die vier Drahtstränge 2a, 2b, 2d, 2e bildenden Drahtstücke. Während im Beispiel der Fig. 3 bis 6 die beiden Drahtstücke überkreuzungsfrei mit ihren U-förmigen Umlenkbereichen 2c, 2f jeweils durch zwei benachbarte der vier Durchführungsöffnungen 14a bis 14d durchgeschleift sind, sind sie im Beispiel von Fig. 11 mit sich überkreuzenden U-förmigen Umlenkbereichen 2c, 2d durch jeweils zwei diametral gegenüberliegende 14a, 14c bzw. 14b, 14d der vier Durchführungsöffnungen 14a bis 14d durchgeschleift. In diesem Fall bildet folglich jedes der beiden Drahtstücke je zwei gegenüberliegende, um 180° in Drahtkorbumfangsrichtung versetzte Drahtstränge von den vier korbbildenden Drahtsträngen 2a, 2b, 2d, 2e. Für gewisse Anwendungsfälle kann diese sich überkreuzende Fixierung der beiden Drahtstücke und damit der vier Drahtstränge 2a, 2b, 2d, 2e am Fixierscheibenelement 13a oder an dem erfindungsgemäßen, gewölbten Fixierscheibenelement (13a, 13b) von Fig. 10, Vorteile bieten.

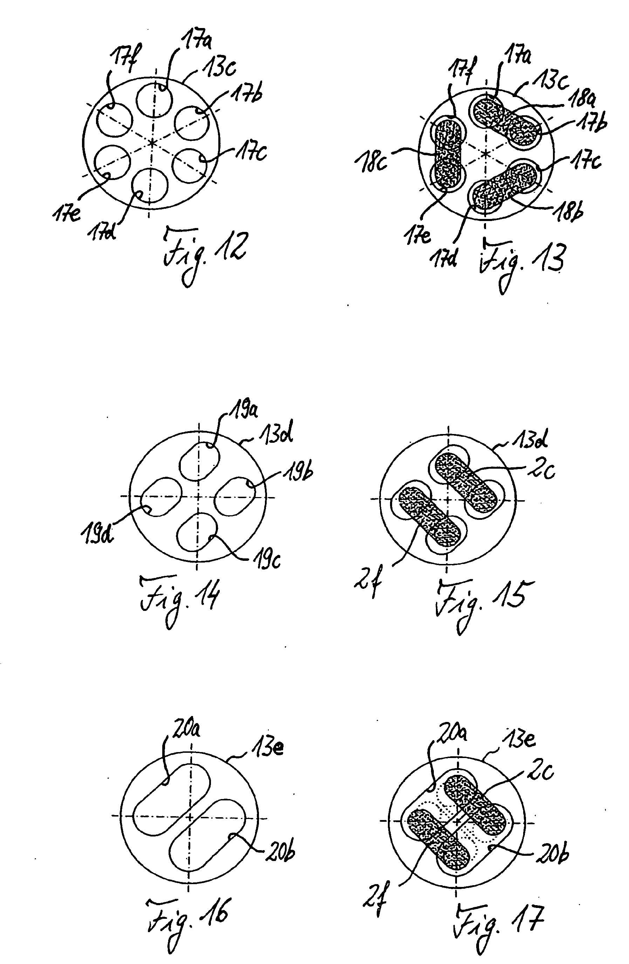

Die Fig. 12 bis 19 veranschaulichen anhand einiger exemplarischer Beispiele die an die Erfordernisse des jeweiligen Anwendungsfalls anpassbare hohe Variabilität hinsichtlich der Gestaltung des erfindungsgemäßen Fixierscheibenelements, wobei für jedes gezeigte Beispiel das betreffende Fixierscheibenelement in Draufsicht von vorne einmal ohne und einmal mit durchgeschleiften Drahtstücken wiedergegeben ist.

Die Fig. 12 und 13 zeigen ein Fixierscheibenelement 13c mit sechs kreisrunden Durchführungsöffnungen 17a bis 17f, die in Umfangswinkelrichtung äquidistant in das Fixierscheibenelement 13c eingebracht sind. Wie aus Fig. 13 ersichtlich, sind drei Drahtstücke mit ihrem U-förmigen Umlenkbereich 18a, 18b, 18c durch jeweils zwei benachbarte Durchführungsöffnungen 17a und 17b, 17c und 17d bzw. 17e und 17f durchgeschleift und bilden dadurch je zwei in Korbumfangsrichtung benachbarte Drahtstränge eines solchermaßen aus sechs Drahtsträngen bestehenden ballonförmigen Drahtkorbs. In entsprechenden Anwendungen ist ein solcher engmaschigerer Drahtkorb mit sechs Drahtsträngen von Vorteil. Es versteht sich, dass auch bei diesem Beispiel eines aus sechs Drahtsträngen aufgebauten Drahtkorbs alternativ zu dem in Fig. 13 gezeigten kreuzungsfreien Durchschleifen der Drahtstücke ein sich überkreuzendes Durchschleifen von wenigstens zwei der drei Drahtstücke analog zum Ausführungsbeispiel von Fig. 11 vorgesehen sein kann, z.B. indem jedes der drei Drahtstücke in je zwei sich diametral gegenüberliegende der sechs Durchführungsöffnungen 17a bis 17f durchgeschleift ist.

Die Fig. 14 und 15 veranschaulichen ein Fixierscheibenelement 13d, das dem Fixierscheibenelement 13a der Fig. 3 bis 6 mit der Modifikation entspricht, dass die Durchführungsöffnungen in einer geringfügig länglichen Form gestaltet sind. Speziell weist das Fixierscheibenelement 13d vier Langlochöffnungen 19a bis 19d auf, deren längere Hauptachse senkrecht zum Verlauf des U-förmigen Umlenkbereichs 2c, 2f der wie im Beispiel der Fig. 3 bis 6 überkreuzungsfrei durchgeschleiften beiden Drahtstücke verläuft. Die leichte Langlochform der Durchführungsöffnungen 19a, 19b kann, wenn gewünscht, die Fixierbeweglichkeit der so fixierten beiden Drahtstücke erhöhen, welche die vier Drahtstränge des Drahtkorbs bilden, die sich in Fig. 15 in nicht gezeigter Weise entsprechend dem Beispiel von Fig. 3 nach oben, nach unten, nach rechts bzw. nach links vom Fixierscheibenelement 13d aus radial nach außen und axial hinten erstrecken.

Die Fig. 16 und 17 veranschaulichen ein Fixierscheibenelement 13e, in das zwei Durchführungsöffnungen 20a, 20b mit ausgeprägterer Langlochform eingebracht sind, wobei hier jede Durchführungsöffnung 20a, 20b zwei Drahtstücke bzw. Drahtstränge aufnimmt. Speziell sind, wie aus Fig. 17 zu erkennen, die U-förmigen Umlenkbereiche 2c, 2f der beiden Drahtstränge, welche die vier korbbildenden Drahtstränge bereitstellen, überkreuzungsfrei nebeneinander durch jede der beiden Langloch-Durchführungsöffnungen 20a, 20b durchgeschleift. Die ausgeprägte Langlochform der beiden Durchführungsöffnungen 20a, 20b kann für entsprechende Anwendungen die Beweglichkeit der Drahtstrangfixierung an diesem Fixierscheibenelement 13e im Vergleich zu den vorstehend beschriebenen Ausführungsbeispielen weiter erhöhen.

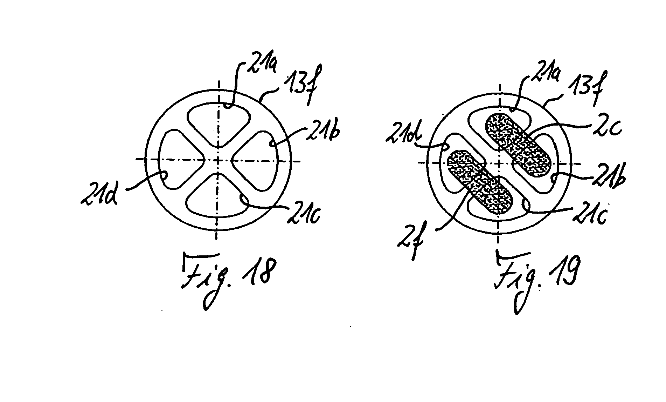

Die Fig. 18 und 19 veranschaulichen ein Fixierscheibenelement 13f, welches wie das Fixierscheibenelement 13a von Fig. 3 vier in Umfangswinkelrichtung äquidistant angeordnete Durchführungsöffnungen 21 a bis 21 d aufweist, die jedoch in diesem Beispiel eine gerundet kreissektorförmige Gestalt haben, wie gezeigt. Wiederum sind wie im Beispiel der Fig. 3 bis 6 die beiden Drahtstücke bzw. die von Ihnen gebildeten vier Drahtstränge mit sich nicht überkreuzenden, parallel nebeneinander liegenden U-förmigen Umlenkbereichen 2c, 2f an diesem Fixierscheibenelement 13f fixiert. Je nach Querschnittsausdehnung der kreissektorförmigen Durchführungsöffnungen 21a bis 21d im Vergleich zum Querschnitt der durchgeschleiften Drahtstücke kann auch in diesem Ausführungsbeispiel ebenso wie in den vorangegangenen Ausführungsbeispielen die Beweglichkeit der Fixierung der Drahtstränge am Fixierscheibenelement 13f in gewünschter Weise eingestellt werden. Auch zum Ausführungsbeispiel der Fig. 18 und 19 ist entsprechend dem Beispiel von Fig. 11 alternativ eine sich überkreuzende Fixierung der Drahtstränge möglich.

Wenngleich die gezeigten Fixierscheibenelemente 13a bis 13f jeweils eine Kreisscheibenform aufweisen, versteht es sich, dass das erfindungsgemäße Fixierscheibenelement je nach Bedarf eine beliebige andere Randform haben kann, z.B. oval oder in Form eines Vielecks oder auch eine unsymmetrische Form. Unabhängig von seiner äußeren Form weist das erfindungsgemäße Fixierscheibenelement nach Art eines Knopfes wenigstens zwei Durchführungsöffnungen zur Fixierung wenigstens eines der korbbildenden Drahtstränge auf. Im allgemeinen sind alle Drahtstränge, die den ballonförmigen Drahtkorb der erfindungsgemäßen Drahtkorbeinheit bilden, auf diese Weise mittels Durchschleifen durch zwei Durchführungsöffnungen unter U-förmiger Umlenkung am Fixierscheibenelement fixiert, dies ist jedoch nicht zwingend. Es können auch Mischformen zur Anwendung kommen, bei denen diese Fixierungsart nur bei einem Teil der den Drahtkorb bildenden Drahtstränge vorgesehen ist. Meist ist es vorteilhaft, in der gezeigten und beschriebenen Weise zwei oder mehr der korbbildenden Drahtstränge durch ein gemeinsames Drahtstück bereitzustellen. Auch dies ist aber nicht zwingend, vielmehr ist es bei Bedarf möglich, dass wenigstens einer der Drahtstränge nach U-förmiger Umlenkung und Durchschleifen durch die zweite zugehörige Durchführungsöffnung an der Rückseite des Fixierscheibenelements endet, z.B. als kugelförmig verdicktes Drahtende, das ihn gegen Herausbewegen aus der Durchführungsöffnung sichert.

Unabhängig von der konkreten Realisierung weist die erfindungsgemäße Drahtkorbeinheit durch die spezifische, oben erläuterte Art der Drahtfixierung am entsprechend mit Durchführungsöffnungen versehenen Fixierscheibenelement einen optimierten vorderen Drahtkorbabschluss des ballonförmigen Drahtkorbs hinsichtlich Beweglichkeit einerseits und Stabilität andererseits auf. In Kombination mit einer Verwendung von hochflexiblem Drahtmaterial für die korbbildenden Drahtstränge ermöglicht die erfindungsgemäße Drahtkorbeinheit ein zuverlässiges und reproduzierbares Zusammenfalten des Drahtkorbs beim Einziehen in eine Aufnahmehülse und umgekehrt ein zuverlässiges und funktionssicheres Auffalten des Drahtkorbs beim Herausbewegen aus der Aufnahmehülse. Dabei wird die Bewegung des Zusammenfaltens bzw. Auffaltens durch die materialspezifische Elastizität der Drahtstränge und die spezifische Drahtstrangfixierung am Fixierscheibenelement unterstützt, die z.B. so gewählt sein kann, dass die korbbildenden Drahtstränge beim Zusammenfalten ebenso wie beim Auffalten jeweils eine leichte Kippbewegung an der Fixierstelle ausführen, wie oben beispielsweise zu Fig. 9 erläutert. Gleichzeitig verhindert die spezifische Drahtstrangfixierung in jeweils zwei Durchführungsöffnungen am Fixierscheibenelement ein zu starkes Verdrehen des Drahtkorbs, was beispielsweise zu einem unerwünschten Abkippen des Drahtkorbs führen könnte.

Wie erwähnt, ist die erfindungsgemäße Drahtkorbeinheit insbesondere in medizinischen Instrumenten einsetzbar, wie in Steinfangkorb- und Polypektomieschlingeninstrumenten, jedoch kann die erfindungsgemäße Drahtkorbeinheit selbstverständlich auch in beliebigen anderen Anwendungen eingesetzt werden, bei denen Bedarf an einem flexiblen, ballonförmigen Drahtkorb besteht.

## Patentansprüche

1. Drahtkorbeinheit, insbesondere für ein medizinisches Drahtkorbinstrument, mit
- einem ballonförmigen Drahtkorb (1) aus mehreren Drahtsträngen (2a, 2b, 2d, 2e), die sich zwischen einem vorderen und einem hinteren Drahtkorbende (3, 4) erstrecken und von einem oder mehreren Drahtstücken gebildet sind, und
- einem Fixierscheibenelement (13a, 13b), an dem die Drahtstränge am vorderen Drahtkorbende unter Bildung eines vorderen Drahtkorbabschlusses fixiert sind, wobei das Fixierscheibenelement wenigstens eine erste und eine zweite Durchführungsöffnung (14a bis 14d) aufweist,
wobei wenigstens ein erster (2a) der Drahtstränge von einem ersten Drahtstück gebildet ist, das durch eine erste (14a) der Durchführungsöffnungen des Fixierscheibenelements (13a, 13b) von hinten nach vorne und unter U-förmiger Umlenkung (2c) durch eine zweite (14b) der Durchführungsöffnungen des Fixierscheibenelements von vorne wieder nach hinten durchgeschleift und so am Fixierscheibenelement fixiert ist,
**dadurch gekennzeichnet, dass**
das Fixierscheibenelement (13a, 13b) eine von einem Umfangsrandbereich (15) zu einem Mittenbereich (16) vorgewölbt gebogene Form aufweist.

2. Drahtkorbeinheit nach Anspruch 1, weiter **dadurch gekennzeichnet, dass** der erste und wenigstens ein zweiter (2b) der Drahtstränge des Drahtkorbs von einem einteiligen ersten Drahtstück gebildet sind, das sich von einem hinteren Ende (4) des Drahtkorbs nach vorne zum Fixierscheibenelement (13a) und durch die erste Durchführungsöffnung (14a) hindurch und von dort unter U-förmiger Umlenkung zurück durch die zweite Durchführungsöffnung (14b) hindurch und bis zum hinteren Drahtkorbende erstreckt.

3. Drahtkorbeinheit nach Anspruch 2, weiter **dadurch gekennzeichnet, dass** mehrere Paare der Drahtstränge von jeweils einem einteiligen Drahtstück gebildet sind, das sich jeweils vom hinteren Drahtkorbende nach vorne zum Fixierscheibenelement, durch eine zugehörige erste Durchführungsöffnung desselben hindurch und von dort unter U-förmiger Umlenkung zurück durch eine zugehörige zweite Durchführungsöffnung hindurch bis zum hinteren Drahtkorbende erstreckt.

4. Drahtkorbeinheit nach einem der Ansprüche 1 bis 3, weiter **dadurch gekennzeichnet, dass** wenigstens eine der Durchführungsöffnungen eine kreisförmige oder langlochförmige oder kreissektorförmige Gestalt aufweist.

5. Drahtkorbeinheit nach einem der Ansprüche 1 bis 4, weiter **dadurch gekennzeichnet, dass** die Durchführungsöffnungen mit gleichem Abstand von einem Mittelpunkt (M) und in Umfangswinkelrichtung des Fixierscheibenelements äquidistant angeordnet sind.

6. Drahtkorbeinheit nach einem der Ansprüche 1 bis 5, weiter **dadurch gekennzeichnet, dass** durch wenigstens eine der Durchführungsöffnungen (20a, 20b) wenigstens zwei Drahtstücke durchgeschleift sind.

7. Drahtkorbeinheit nach einem der Ansprüche 1 bis 6, weiter **dadurch gekennzeichnet, dass** die Drahtstränge überkreuzungsfrei am Fixierscheibenelement fixiert sind.

8. Drahtkorbeinheit nach einem der Ansprüche 1 bis 7, weiter **dadurch gekennzeichnet, dass** wenigstens das erste Drahtstück mit seinem U-förmigen Umlenkbereich (2c) zwischen der ersten und der zweiten Durchführungsöffnung (14a, 14b) an der Vorderseite des Fixierscheibenelements in einer Längsebene verläuft, die nicht-parallel zu einer Längsebene ist, in welcher der erste Drahtstrang (2a) im korbbildenden Bereich zwischen dem vorderen und dem hinteren Drahtkorbende (3, 4) verläuft.

## Claims

1. Wire basket unit, in particular for a medical wire basket instrument, with
- a balloon-shaped wire basket (1) composed of several wire strands (2a, 2b, 2d, 2e) which extend between a front end (3) of the wire basket and a rear end (4) of the wire basket and are formed from one or more wire segments, and
- a fixing disc element (13a, 13b) on which the wire strands are fixed at the front end of the wire basket so as to form a front closure of the wire basket, wherein the fixing disc element has at least a first through-opening and a second through-opening (14a to 14d),
wherein at least a first (2a) of the wire strands is formed from a first wire segment which is looped through a first (14a) of the through-openings of the fixing disc element (13a, 13b) from the rear forwards and, forming a U-shaped deflection (2c), is looped through a second (14b) of the through-openings of the fixing disc element from the front to the rear again and is thus fixed on the fixing disc element, **characterized in that**
the fixing disc element (13a, 13b) has a shape that curves forwards from a circumferential edge area (15) to a central area (16).

2. Wire basket unit according to Claim 1, further **characterized in that** the first and at least a second (2b) of the wire strands of the wire basket are formed from a one-piece first wire segment which extends forwards from a rear end (4) of the wire basket to the fixing disc element (13a) and through the first through-opening (14a) and from there, forming a U-shaped deflection, extends back through the second through-opening (14b) as far as the rear end of the wire basket.

3. Wire basket unit according to Claim 2, further **characterized in that** several pairs of the wire strands are formed in each case from a one-piece wire segment which in each case extends forwards from the rear end of the wire basket to the fixing disc element, through an associated first through-opening thereof, and from there, forming a U-shaped deflection, extends back through an associated second through-opening as far as the rear end of the wire basket.

4. Wire basket unit according to one of Claims 1 to 3, further **characterized in that** at least one of the through-openings is formed in the shape of a circle or in the shape of an oblong hole or in the shape of a sector of a circle.

5. Wire basket unit according to one of Claims 1 to 4, further **characterized in that** the through-openings are arranged at the same distance from a centre point (M) and are equidistant in the circumferential direction of the fixing disc element.

6. Wire basket unit according to one of Claims 1 to 5, further **characterized in that** at least two wire segments are looped through at least one of the through-openings (20a, 20b).

7. Wire basket unit according to one of Claims 1 to 6, further **characterized in that** the wire strands are fixed on the fixing disc element without intersecting.

8. Wire basket unit according to one of Claims 1 to 7, further **characterized in that** at least the first wire segment with its U-shaped deflection area (2c) between the first and second through-openings (14a, 14b) on the front side of the fixing disc element extends in a longitudinal plane which is non-parallel to a longitudinal plane in which the first wire strand (2a) extends in the basket-forming area between the front end (3) and rear end (4) of the wire basket.

## Revendications

1. Unité de panier en fils métalliques, en particulier pour un instrument médical équipé d'un panier en fils métalliques, comprenant
- un panier en fils métalliques en forme de ballonnet (1) constitué de plusieurs câbles métalliques (2a, 2b, 2d, 2e) qui s'étendent entre une extrémité avant et une extrémité arrière du panier en fils métalliques (3, 4) et qui sont formés par un ou plusieurs brins métalliques, et
- un élément de disque de fixation (13a, 13b) au niveau duquel les câbles métalliques sont fixés à l'extrémité avant du panier en fils métalliques en formant une terminaison avant de panier en fils métalliques, l'élément de disque de fixation présentant au moins une première et une deuxième ouverture de passage (14a à 14d),
au moins un premier (2a) des câbles métalliques étant formé par un premier brin métallique, qui est enfilé à travers une première (14a) des ouvertures de passage de l'élément de disque de fixation (13a, 13b) de l'arrière vers l'avant et par un renvoi en forme de U (2c) à travers une deuxième (14b) des ouvertures de passage de l'élément de disque de fixation de l'avant à nouveau vers l'arrière et est ainsi fixé à l'élément de disque de fixation,
**caractérisée en ce que**
l'élément de disque de fixation (13a, 13b) présente une forme courbe précintrée depuis une région de bord périphérique (15) vers une région centrale (16).

2. Unité de panier en fils métalliques selon la revendication 1, **caractérisée en outre en ce que** le premier et au moins un deuxième (2b) des câbles métalliques du panier en fils métalliques sont formés par un premier brin métallique d'une seule pièce qui s'étend depuis une extrémité arrière (4) du panier en fils métalliques vers l'avant jusqu'à l'élément de disque de fixation (13a) et à travers la première ouverture de passage (14a) et de là, par un renvoi en forme de U, revient à travers la deuxième ouverture de passage (14b) et s'étend jusqu'à l'extrémité arrière du panier en fils métalliques.

3. Unité de panier en fils métalliques selon la revendication 2, **caractérisée en outre en ce que** plusieurs paires des câbles métalliques sont formées à chaque fois par un brin métallique d'une seule pièce qui s'étend à chaque fois depuis l'extrémité arrière du panier en fils métalliques vers l'avant jusqu'à l'élément de disque de fixation, à travers une première ouverture de passage associée de celui-ci, et de là, par un renvoi en forme de U, revient à travers une deuxième ouverture de passage associée jusqu'à l'extrémité arrière du panier en fils métalliques.

4. Unité de panier en fils métalliques selon l'une quelconque des revendications 1 à 3, **caractérisée en outre en ce qu'**au moins l'une des ouvertures de passage présente une forme circulaire ou une forme de trou oblong ou une forme de secteur circulaire.

5. Unité de panier en fils métalliques selon l'une quelconque des revendications 1 à 4, **caractérisée en outre en ce que** les ouvertures de passage sont disposées à la même distance d'un centre (M) et de manière équidistante dans la direction angulaire périphérique de l'élément de disque de fixation.

6. Unité de panier en fils métalliques selon l'une quelconque des revendications 1 à 5, **caractérisée en outre en ce qu'**au moins deux brins métalliques sont enfilés à travers au moins l'une des ouvertures de passage (20a, 20b).

7. Unité de panier en fils métalliques selon l'une quelconque des revendications 1 à 6, **caractérisée en outre en ce que** les câbles métalliques sont fixés sans croisements à l'élément de disque de fixation.

8. Unité de panier en fils métalliques selon l'une quelconque des revendications 1 à 7, **caractérisée en outre en ce qu'**au moins le premier brin métallique s'étend avec sa région de renvoi en forme de U (2c) entre la première et la deuxième ouverture de passage (14a, 14b) au niveau du côté avant de l'élément de disque de fixation dans un plan longitudinal qui n'est pas parallèle à un plan longitudinal dans lequel le premier câble métallique (2a) s'étend dans la région formant le panier entre l'extrémité avant et l'extrémité arrière du panier en fils métalliques (3, 4).
